Europäisches Patentamt
European Patent Office
Office européen des brevets

(19)

(11) Publication number: **0 155 810**
**B1**

(12) # EUROPEAN PATENT SPECIFICATION

(45) Date of publication of patent specification: **05.07.89**

(21) Application number: **85301696.2**

(22) Date of filing: **12.03.85**

(51) Int. Cl.4: **C 07 D 303/22,** C 07 D 303/27, C 07 D 303/36 // C09J3/16

(54) **N-(omega-glycidoxyalkyl)-substituted amide compounds and preparation process thereof.**

(30) Priority: **15.03.84 JP 48185/84**

(43) Date of publication of application: **25.09.85 Bulletin 85/39**

(45) Publication of the grant of the patent: **05.07.89 Bulletin 89/27**

(84) Designated Contracting States: **CH DE FR GB IT LI NL SE**

(56) References cited:
**EP-A-0 094 436**
**GB-A-1 170 837**
**US-A-3 244 731**

(73) Proprietor: **MITSUI TOATSU CHEMICALS, INCORPORATED**
**2-5 Kasumigaseki 3-chome**
**Chiyoda-Ku Tokyo 100 (JP)**

(72) Inventor: **Itoh, Hiroshi**
**521, Kasamacho Totsuka-ku**
**Yokohama Kanagawa-ken (JP)**
Inventor: **Tanaka, Tomio**
**8-14-2, Aoto**
**Katsushika-ku Tokyo (JP)**
Inventor: **Nitta, Atsuhiko**
**634-1-154, Nobacho Kohnan-ku**
**Yokohama Kanagawa-ken (JP)**
Inventor: **Kamio, Hideo**
**728, Sogabetsusho**
**Odawara Kanagawa-ken (JP)**

(74) Representative: **Armitage, Ian Michael et al**
**MEWBURN ELLIS & CO. 2/3 Cursitor Street**
**London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel N-(ω-glycidoxyalkyl)-substituted amide compounds and their preparation process.

Glycidyl-substituted compounds have already found wide-spread commercial utility as raw materials for epoxy resins, fixing base materials for various functional materials, etc. More specifically, there have been known those having such skeletal structures as glycidyl ethers, glycidyl amines and glycidyl esters. Recently, a variety of N-glycidyl-substituted amide compounds have been synthesized by the present inventors, thereby demonstrating the existence of such novel compounds (see, Japanese Patent Laid-open Nos. 88373/1983 and 16883/1984). In each of such novel compounds, the glycidyl group is however bonded adjacent to the skeletal structure. They are thus accompanied by such drawbacks that when used as epoxy resins, their hardened bodies are brittle and when used as fixing base materials, their fixing efficiency decreases as the molecular size of a functional compound to be fixed increases.

On the other hand, N-substituted amide derivatives have some characteristic properties unseen in the other skeletal structures, namely, they have better compatibility with various substances and are less susceptible to hydrolyses compared with corresponding ether-, amine- and ester-derivatives.

With the foregoing in view, the present inventors have carried out an extensive research with a view toward developing a conventionally-unknown, novel bonding pattern for glycidyl group while making use of an amido group as a skeletal structure. As a result, they have found novel N-(ω-glycidoxyalkyl)-substituted amide compounds, in each of which an amido group and glycidyl group are bonded together by way of a polymethylenoxy group, and their preparation process, leading to the present invention.

This invention relates to N-(ω-glycidoxyalkyl)-substituted amide compounds and their preparation process. More specifically, it relates to N-substituted amide compounds in which the amido groups of amide compounds have been substituted by ω-glycidoxyalkyl groups as well as their preparation process.

The above N-(ω-glycidoxyalkyl)-substituted amide compounds can be obtained by reacting amide compounds and halogen-substituted glycidyl ether compounds in the presence of a strong basic substance in an aprotic polar solvent.

The N-(ω-glycidoxyalkyl)-substituted amide compounds of this invention are suitable for use in the production of adhesives and the like.

The compounds of this invention are N-(ω-glycidoxyalkyl)-substituted amide compounds represented by the following general formula:

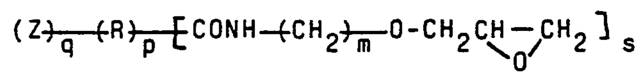

(1a)

or

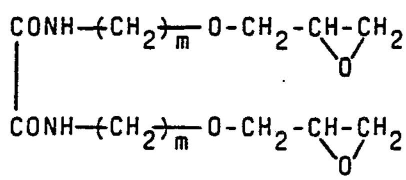

(1b)

wherein R means an aliphatic, aromatic or alicyclic hydrocarbon group, Z denotes a hydrogen atom or an ω-glycidoxyalkoxy or N,N-di(ω-glycidoxyalkyl)amino group, p stands for an integer of 0 or 1, q is an integer of 1—4, and s is an integer of 1—4 and m is an integer of 4—20, with the provisos that: (i) q is 1, Z is H and s is 1 when p = 0, and (ii) p is 1 when Z denotes an ω-glycidoxyalkoxy or N,N-di(ω-glycidoxyalkyl)amino group. They are novel compounds not reported in literatures.

The compounds of this invention can be prepared by reacting an amide compound represented by the following general formula:

$$(Y)_q\text{—}(R)_p\text{—}(CONH_2)_s \qquad (2)$$

wherein Y denotes a hydrogen atom or a hydroxyl or amino group and R is as defined above, p = q = 0, and s = 2; or R, q, p, and s are as defined above with the proviso that q is 1, Y is H and s is 1 when p is 0, with a halogen-substituted glycidyl ether compound represented by the following general formula:

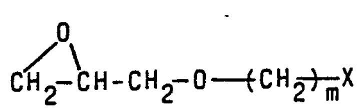

(3)

wherein X means a halogen atom and m has the same meaning as defined above, in the presence of a strong basic substance in an aprotic polar solvent.

The compounds of this invention, which are represented by the general formula (1), may be classified into N-(ω-glycidoxyalkyl)-substituted compounds of monoamide compounds, N-(ω-glycidoxyalkyl)-substituted compounds of polyamide compounds including diamide compounds, N-(ω-glycidoxyalkyl)-substituted ω-glycidoxyalkyl ether compounds of hydroxyl-substituted monoamide compounds, N-(ω-glycidoxyalkyl)-substituted ω-glycidoxyalkyl ether compounds of hydroxyl-substituted diamide compounds, N-(ω-glycidoxyalkyl)-substituted compounds of amino-substituted monoamide compounds, N-(ω-glycidoxyalkyl)-substituted compounds of amino-substituted diamide compounds, etc.

N-(ω-Glycidoxyalkyl)-substituted amide compounds of monoamide compounds are represented by the following general formula (4):

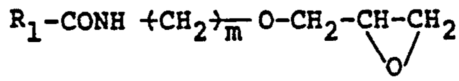
(4)

wherein $R_1$ means an alkyl, alkenyl, aryl or alicyclic group. The alkyl group is represented by the general formula $C_nH_{2n+1}$— in which n stands for an integer of 0—20. The alkenyl group is represented by the general formula $C_nH_{2n-1}$— in which n stands for an integer of 2—20. The aryl group is a substituent group containing an aromatic ring and may also include an arylalkyl or arylalkenyl group. As the aromatic ring, a benzene ring, naphthalene ring, anthracene ring or the like may be applied. The alicyclic ring is a substituent group which contains an alicyclic structure.

The compounds of this invention also include those containing the above-described substituent groups in each of which one or more halogen atoms, which may be of the same type or of different types, have been introduced to the hydrocarbyl moiety of the respective substituent group. m stands for a positive integer of 4—20.

Among polyamide compounds, N-(ω-glycidoxyalkyl)-substituted amide compounds of diamide compounds are represented by the following general formula (5):

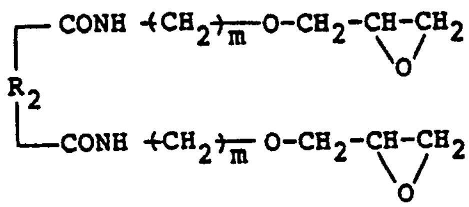
(5)

In the general formula (5), $R_2$ means an alkylene, alkenylene, arylene or alicyclic group. The alkylene group is represented by the general formula —$C_nH_{2n}$— in which n stands for an integer of 0—20. The alkenylene group is represented by the general formula —$C_nH_{2n-2}$— in which n stands for an integer of 2—20. The arylene group is a substituent group containing an aromatic ring and may include an arylalkyl or arylalkenyl group. As the aromatic ring, a benzene ring, naphthalene ring, anthracene ring or the like may be applied. The alicyclic group has a structure in which an alicyclic structure is contained.

The compounds of this invention also include those containing the above-described substituent groups in each of which one or more halogen atoms, which may be of the same type or of different types, have been introduced to the hydrocarbyl moiety of the respective substituent group. m stands for a positive integer of 4—20.

N-(ω-glycidoxyalkyl)-substituted amide compounds of triamide compounds are represented by the following general formula (6):

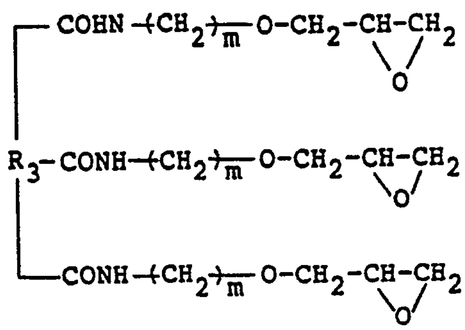
(6)

In the general formula (6), $R_3$ means an aromatic or alicyclic ring. As the aromatic ring, a benzene ring, naphthalene ring, anthracene ring or the like may be applied. Also included are those containing one or more halogen atoms, which may be of the same type or different types, substituted to an aromatic ring. m is a positive integer of 4—20.

N-(ω-Glycidoxyalkyl)-substituted amide compounds of tetraamide compounds are represented by the following general formula (7):

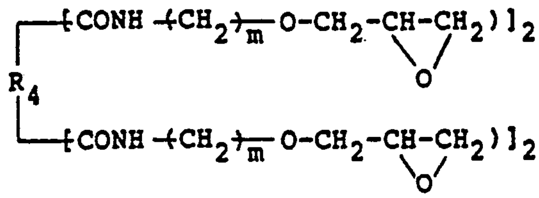

(7)

In the general formula (7), $R_4$ means an aromatic or alicyclic ring. As the aromatic ring, a benzene ring, naphthalene ring, anthracene ring or the like may be applied. Also included are those containing one or more halogen atoms, which may be of the same type or different types, substituted to an aromatic ring. m stands for a positive integer of 4—20.

N-(ω-Glycidoxyalkyl)-substituted ω-glycidoxyether compounds of hydroxyl-substituted monoamide compounds are represented by the following general formula (8):

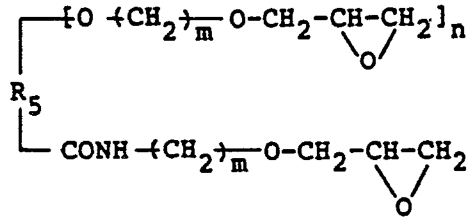

(8)

In the above formula, n stands for an integer of 1—4. When n = 1, $R_5$ is an alkylene, alkenylene or arylene group. When n = 2—4, $R_5$ indicates an aromatic ring and a benzene ring, naphthalene ring, anthracene ring or the like may be applied.

The alkylene group is rpresented by the general formula $—C_nH_{2n}—$ in which n is an integer of 1—20. The alkenylene group is represented by the general formula $—C_nH_{2n-2}$ in which n stands for an integer of 2—20. The arylene group is a substituted group containing an aromatic ring. As the aromatic ring, a benzene ring, naphthalene ring, anthracene ring or the like may be applied. Also included are those containing one or more halogen atoms, which may be of the same type or of different types, substituted to the hydrocarbyl portions of the above substituent groups. m stands for a positive integer of 4—20.

N-(ω-Glycidoxyalkyl)-substituted ω-glycidoxyalkyl ether compounds of hydroxyl-substituted diamide compounds are represented by the following general formula (9):

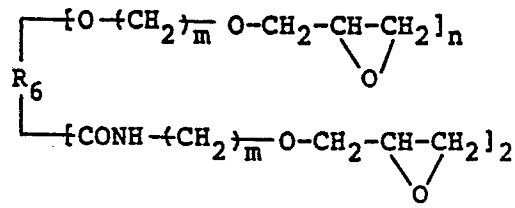

(9)

In the general formula (9), n is an integer of 1 or 2. $R_6$ means an aromatic ring, and a benzene ring, naphthalene ring, anthracene ring or the like may be applied. Also included are those containing one or more halogen atoms, which may be of the same type or of different types, substituted to the hydrocarbyl moieties of the substituent groups.

N-(ω-Glycidoxyalkyl)-substituted amide compounds of amino-substituted monoamide compounds are represented by the following general formula (10):

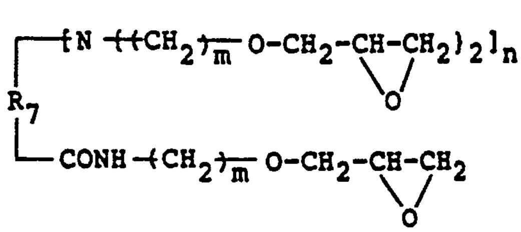

(10)

In the general formula (10), n stands for an integer of 1 or 2. $R_7$ means an alkylene, arylene or alicyclic group. The alkylene group is represented by the general formula $—C_nH_{2n}—$ in which n stands for an integer of 1—20. The arylene group is a substituent group containing an aromatic ring and may include an arylalkyl

group. As the aromatic ring, a benzene ring, naphthalene ring, anthracene ring or the like may be applied. The alicyclic ring is a substituent group which has an alicyclic structure. Also included are those containing one or more halogen atoms, which may be of the same type or of different types, substituted to the hydrocarbyl moieties of the above substituent groups. m stands for a positive integer of 4—20.

N-(ω-Glycidoxyalkyl)-substituted amide compounds of amino-substituted diamide compounds are represented by the following general formula (11):

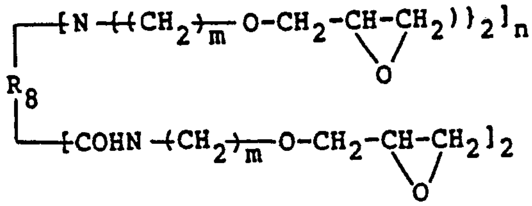

(11)

In the general formula (11), n stands for an integer of 1 or 2. $R_8$ means an aromatic ring. As the aromatic ring, a benzene ring, naphthalene ring, anthracene ring or the like may be applied. Also included are those containing one or more halogen atoms, which may be of the same type or of different types, substituted to the aromatic ring. m stands for a positive integer of 4—20.

Certain representative examples of the compounds of this invention will hereinafter be given by way of example. Since the present invention includes an extremely wide variety of compounds therein, exemplification will first of all be made by fixing m at 4 in the general formula (1) and changing R, Z, p, q and s in various ways in order to simplify the exemplification of compounds. Thereafter, further exemplification will be made by fixing R at a vinylene group (—CH=CH—), Z at a hydrogen atom, and p, q and s at 1 and changing m to 4—20.

As exemplary N-(ω-glycidoxyalkyl)-substituted amide compounds of monoamide compounds, may be mentioned:

N-(4-glycidoxybutyl)formamide;
N-(4-glycidoxybutyl)acetamide;
N-(4-glycidoxybutyl)propionamide;
N-(4-glycidoxybutyl)butyramide;
N-(4-glycidoxybutyl)chloroacetamide;
N-(4-glycidoxybutyl)chloropropionamide;
N-(4-glycidoxybutyl)dichloroacetamide;
N-(4-glycidoxybutyl)acrylamide;
N-(4-glycidoxybutyl)methacylamide;
N-(4-glycidoxybutyl)crotonamide;
N-(4-glycidoxybutyl)vinylacetamide;
N-(4-glycidoxybutyl)decenamide;
N-(4-glycidoxybutyl)nonadecenamide;
N-(4-glycidoxybutyl)chloroacrylamide;
N-(4-glycidoxybutyl)benzamide;
N-(4-glycidoxybutyl)naphthalenecarboxamide;
N-(4-glycidoxybutyl)anthracenecarboxamide;
N-(4-glycidoxybutyl)tolylamide;
N-(4-glycidoxybutyl)phenylacetamide;
N-(4-glycidoxybutyl)phenylpropionamide;
N-(4-glycidoxybutyl)phenylacrylamide;
N-(4-glycidoxybutyl)benzylacrylamide;
N-(4-glycidoxybutyl)cinnamide;
N-(4-glycidoxybutyl)allylbenzamide;
N-(4-glycidoxybutyl)chlorobenzamide;
N-(4-glycidoxybutyl)dichlorobenzamide;
N-(4-glycidoxybutyl)chlorobromobenzamide;
N-(4-glycidoxybutyl)cyclobutanecarboxamide;
N-(4-glycidoxybutyl)cyclopentanecarboxamide;
N-(4-glycidoxybutyl)cyclohexanecarboxamide;
N-(4-glycidoxybutyl)cycloheptanecarboxamide;
N-(4-glycidoxybutyl)cyclohexylacetamide;
N-(4-glycidoxybutyl)cyclohexylpropionamide;
N-(4-glycidoxybutyl)cyclohexenecarboxamide;
N-(4-glycidoxybutyl)cyclohexadienecarboxamide; etc.

Illustrative N-(ω-glycidoxyalkyl)-substituted amide compounds of polyamide compounds may include:

N,N'-di(4-glycidoxybutyl)oxamide;
N,N'-di(4-glycidoxybutyl)malonamide;
N,N'-di(4-glycidoxybutyl)succinimide;
N,N'-di(4-glycidoxybutyl)glutaramide;
N,N'-di(4-glycidoxybutyl)adipamide;
N,N'-di(4-glycidoxybutyl)piperamide;
N,N'-di(4-glycidoxybutyl)suberamide;
N,N'-di(4-glycidoxybutyl)azelamide;
N,N'-di(4-glycidoxybutyl)sebacamide;
N,N'-di(4-glycidoxybutyl)octadecanedicarboxamide;
N,N'-di(4-glycidoxybutyl)fumaramide;
N,N'-di(4-glycidoxybutyl)maleamide;
N,N'-di(4-glycidoxybutyl)citraconamide;
N,N'-di(4-glycidoxybutyl)mesaconamide;
N,N'-di(4-glycidoxybutyl)decenedicarboxamide;
N,N'-di(4-glycidoxybutyl)octadecene-dicarboxamide;
N,N'-di(4-glycidoxybutyl)phthalamide;
N,N'-di(4-glycidoxybutyl)isophthalamide;
N,N'-di(4-glycidoxybutyl)terephthalamide;
N,N'-di(4-glycidoxybutyl)naphthalenedicarboxamide;
N,N'-di(4-glycidoxybutyl)anthracenedicarboxamide;
N,N'-di(4-glycidoxybutyl)carbamoylphenylacetamide;
N,N'-di(4-glycidoxybutyl)phenylcitraconamide;
N,N'-di(4-glycidoxybutyl)diphenamide;
N,N'-di(4-glycidoxybutyl)chloroisophthalamide;
N,N'-di(4-glycidoxybutyl)bromoisophthalamide;
N,N'-di(4-glycidoxybutyl)cyclobutanedicarboxamide;
N,N'-di(4-glycidoxybutyl)cyclopentanedicarboxamide;
N,N'-di(4-glycidoxybutyl)cyclohexane dicarboxamide;
N,N'-di(4-glycidoxybutyl)cycloheptanedicarboxamide;
N,N'-di(4-glycidoxybutyl)cyclohexenedicarboxamide;
N,N'-di(4-glycidoxybutyl)camphoramide;
N,N',N''-tri(4-glycidoxybutyl)benzenetricarboxamide;
N,N',N''-tri(4-glycidoxybutyl)naphthalenetricarboxamide;
N,N',N''-tri(4-glycidoxybutyl)toluenetricarboxamide;
N,N',N''-tri(4-glycidoxybutyl)chlorobenzenetricarboxamide;
N,N',N''-tri(4-glycidoxybutyl)cyclohexanetricarboxamide;
N,N',N'',N'''-tetra(4-glycidoxybutyl)pyromellitamide;
N,N',N'',N'''-tetra(4-glycidoxybutyl)naphthalenetetracarboxamide;
N,N',N'',N'''-tetra(4-glycidoxybutyl)cycloheptanetetracarboxamide;
etc.

Exemplary N-(ω-glycidoxyalkyl)-substituted ω-glycidoxyalkyl ether compounds of hydroxyl-substituted monoamide compounds may include:

N-(4-glycidoxybutyl)-(4-glycidoxybutoxy)propionamide;
N-(4-glycidoxybutyl)-(4-glycidoxybutoxy)butyramide;
N-(4-glycidoxybutyl)-(4-glycidoxybutoxy)heptanamide;
N-(4-glycidoxybutyl)-(4-glycidoxybutoxy)decanamide;
N-(4-glycidoxybutyl)-(4-glycidoxybutoxy)crotonamide;
N-(4-glycidoxybutyl)-(4-glycidoxybutoxy)dimethylheptynamide;
N-(4-glycidoxybutyl)-(4-glycidoxybutoxy)benzamide;
N-(4-glycidoxybutyl)-(4-glycidoxybutoxy)tolylamide;
N-(4-glycidoxybutyl)-(4-glycidoxybutoxy)phenylbenzamide;
N-(4-glycidoxybutyl)-(4-glycidoxybutoxy)naphthalenecarboxamide;
N-(4-glycidoxybutyl)-di(4-glycidoxybutoxy)benzamide;
N-(4-glycidoxybutyl)-tri(4-glycidoxybutoxy)benzamide
N-(4-glycidoxybutyl-di(4-glycidoxybutoxy)tolylamide;
etc.

As N-(ω-diglycidoxyalkyl)-substituted ω-glycidoxyalkyl ether compounds of hydroxyl-substituted diamide compounds, may for example be mentioned:

N,N'-di(4-glycidoxybutyl)-(4-glycidoxybutoxy)phthalamide;
N,N'-di(4-glycidoxybutyl)-(4-glycidoxybutoxy)isophthalamide;
N,N'-di(4-glycidoxybutyl)-(4-glycidoxybutoxy)terephthalamide;
N,N'-di(4-glycidoxybutyl)-di(4-glycidoxybutoxy)phthalamide;

N,N'-di(4-glycidoxybutyl)-di(4-glycidoxybutoxy)isophthalamide;
N,N'-di(4-glycidoxybutyl)-di(4-glycidoxybutoxy)terephthalamide;
etc.

Illustrative N-(ω-glycidoxyalkyl)-substituted compounds of amino-substituted monoamide compounds may include:
N,N-di(4-glycidoxybutyl)amino-N'-(4-glycidoxybutyl)acetamide;
N,N-di(4-glycidoxybutyl)amino-N'-(4-glycidoxybutyl)propionamide;
N,N-di(4-glycidoxybutyl)amino-N'-(4-glycidoxybutyl)butanamide;
N,N,N',N'-tetra(4-glycidoxybutyl)diamino-N''-(4-glycidoxybutyl)propionamide;
N,N,-di(4-glycidoxybutyl)amino-N'-(4-glycidoxybutyl)benzamide;
N,N-di(4-glycidoxybutyl)amino-N'-(4-glycidoxybutyl)chlorobenzamide;
N,N,-di(4-glycidoxybutyl)amino-N'-(4-glycidoxybutyl)tribromobenzamide;
N,N,N',N'-tetra(4-glycidoxybutyl)diamino-N''-(4-glycidoxybutyl)benzamide;
N,N,-di(4-glycidoxybutyl)aminophenyl-N'-(4-glycidoxybutyl)acetamide;
N,N,-di(4-glycidoxybutyl)aminophenyl-N'-(4-glycidoxybutyl)propionamide;
N,N,-di(4-glycidoxybutyl)amino-N'-(4-glycidoxybutyl)naphthalenecarboxamide;
N,N,-di(4-glycidoxybutyl)amino-N'-(4-glycidoxybutyl)cyclohexanecarboxamide;
etc.

As N-(ω-glycidoxyalkyl)-substituted compounds of amino-substituted diamide compounds, may for example be mentioned:

N,N-di(4-glycidoxybutyl)amino-N',N''-di(4-glycidoxybutyl)succinamide;
N,N-di(4-glycidoxybutyl)amino-N',N''-di(4-glycidoxybutyl)glutardiamide;
N,N-di(4-glycidoxybutyl)amino-N',N''-di(4-glycidoxybutyl)phthalamide;
N,N-di(4-glycidoxybutyl)amino-N',N''-di(4-glycidoxybutyl)isophthalamide;
N,N-di(4-glycidoxybutyl)amino-N',N''-di(4-glycidoxybutyl)terephthalamide;
etc.

Further exemplification by fixing R at a vinylene group (—CH=CH—), Z at a hydrogen atom, and p, q and s respectively at 1 and changing m to 4—20 in the above general formula (1), may for example be mentioned:
N-(4-glycidoxybutyl)acrylamide;
N-(5-glycidoxypentyl)acrylamide;
N-(6-glycidoxyhexyl)acrylamide;
N-(8-glycidoxyoctyl)acrylamide;
N-(10-glycidoxydecyl)acrylamide;
N-(12-glycidoxydodecyl)acrylamide;
N-(14-glycidoxytetradecyl)acrylamide;
N-(16-glycidoxyhexadecyl)acrylamide;
N-(18-glycidoxyoctadecyl)acrylamide;
N-(20-glycidoxyeicosyl)acrylamide;
etc.

The above-exemplified N-(ω-glycidoxyalkyl)substituted amide compounds may be prepared by reacting the amide represented by the general formula (2) with the halogen-substituted glycidyl ether compounds represented by the general formula (3) in the presence of a strong basic substance in an aprotic polar solvent.

The structures of the amide compounds and halogen-substituted glycidyl ether compounds, both of which are useful in the practice of the process of this invention, are exactly the same as the general formula described in detail in connection with the N-(ω-glycidoxyalkyl)-substituted amide compounds and are thus not described in detail here. As the amide compounds, are included those containing aliphatic hydrocarbyl groups, alicyclic groups, aromatic groups or their ring structures as their skeletal structures with 1—4 amide groups introduced therein and optionally one or more hydroxyl or amino groups introduced additionally therein. As the halogen-substituted glycidyl ether compounds, are included those obtained by substituting glycidoxy groups and halogen atoms to both terminal groups of polymethylene chains of 4—20 carbon atoms.

As a specific method for conducting the reaction in the presence of the strong basic substance in the present invention, there are two methods, one being to dissolve the strong basic substance in the liquid reaction mixture and then to initiate the reaction and the other to initiate the reaction with the strong basic substance being in a suspended state. Although both methods may be applicable in the present invention, it has surprisingly been found that an initiation of the reaction in the presence of the strong basic substance in a suspended state is preferred from the viewpoint of the reaction efficiency such as suppression to side reactions. As a more specific method for initiating the reaction in the presence of the strong basic substance in such a suspended state, it may be feasible to employ any suitable method, for example, to charge all the three reaction materials into the aprotic polar solvent and then to mix them there to suspend

the strong basic substance therein; to suspend the strong basic substance in the aprotic polar solvent and then to charge the amide compounds and halogen-substituted glycidyl ether compounds simultaneously to the resulting suspension for their reaction; or to either dissolve or suspend the amide compounds and halogen-substituted glycidyl ether compounds in the aprotic polar solvent and thereafter to add and suspend the strong basic substance.

Any aprotic polar solvent may be employed as a reaction solvent in the present invention. As exemplary aprotic polar solvents, may be mentioned acetonitrile, N,N-dimethylformamide, N,N-dimethylacetamide, dimethylsulfoxide, sulforan, tetraglime, dioxane, N-methylpyrrolidone, and so on.

In a reaction system in which the process of this invention is effected, it is preferred to initiate the reaction in such a state that at least part of the strong basic substance is suspended. The amount of water in such a state is generally 6 wt.% or so as the water quantity in the reaction system. If the amount of water should exceed this level, side reactions such as hydrolysis of the dihalogen-substituted compound tend to occur and the yield will be lowered significantly. In order to allow the reaction to proceed efficiently and hence to increase the yield of the intended product, it is preferred to control the water content of the reaction system below 5 wt.%.

No particular limitation is imposed on the amount of a solvent to be used. The solvent may amount to 5—95 wt.% or preferably 10—90 wt.% of the total weight of reaction materials which include the solvent.

The strong basic substance to be employed in the present invention is a solid substance. Any strong basic substances may be used so long as when either dissolved or suspeded in water, the pHs of resulting aqueous solutions are above 10 or preferably above 11. As such basic substances, may for example be mentioned alkali metal hydroxides, alkali metal oxides, alkali metal carbonates, alkaline earth metal hydroxides, alkaline earth metal oxides, alkali metal hydrides, alkaline earth metal hydrides, alkali metal amides, alkali metal alkoxides, etc. The following specific substances may be given by way of example as the above-described substances. Illustrative of alkali metal hydroxides include sodium hydroxide, potassium hydroxide, lithium hydroxide, rubidium hydroxide and cesium hydroxide. As alkali metal oxides, may for example be mentioned sodium oxide, potassium oxide, lithium oxide, rubidium oxide and cesium oxide. Exemplary alkali metal carbonates may include sodium carbonate, potassium carbonate, lithium carbonate, rubidium carbonate and cesium carbonate. Illustrative of alkaline earth metal hydroxides may include beryllium hydroxide, magnesium hydroxide, calcium hydroxide, strontium hydroxide and barium hydroxide. As alkaline earth metal oxides, may for example be mentioned beryllium oxide, magnesium oxide, calcium oxide, strontium oxide and barium oxide.

Exemplary alkali metal hydrides may include sodium hydride, potassium hydride and lithium hydride. As exemplary alkaline earth metal hydrides, may be mentioned beryllium hydride, magnesium hydride and calcium hydride. Alkali metal amides are compounds obtained by substituting ammonia with alkali metals, including for example sodium amide, potassium amide and lithium amide. Alkali metal alkoxides are compounds obtained by substituting the protons of the hydroxyl groups of alcohols with alkali metals, including for example, sodium methoxide, sodium ethoxide, sodium t-butoxide, potassium methoxide, potassium ethoxide, potassium t-butoxide.

Among the above-described basic substances, those suitable in the practice of the process of this invention may for example be alkali metal hydroxides, alkaline earth metal hydroxides, alkali metal oxides, alkaline earth metal oxides, alkali metal carbonates and the like.

Each of these strong basic substances is usually provided in a solid form for the reaction. The reaction is initiated in such a state that at least part of the strong basic substance is suspended in the liquid reaction mixture.

In the practice of the present invention, the relative amounts of the raw materials, i.e., the amide compound, halogen-substituted glycidyl ether compound and strong basic substance to be used vary depending on the reactivity between the amide compound and the halogen-substituted glycidyl ether compound, etc. and cannot thus be specified as any constant amounts or amount ranges.

However, the halogen-substituted glycidyl ether compound may generally be used in a molar amount 0.2—20 times or preferably 0.3—10 times the amide compound. On the other hand, the strong basic substance may approximately be used in a molar amount 0.2—15 times or preferably 0.5—10 times the amide compound.

A polymerization inhibitor may also be added in order to avoid polymerization of raw materials and reaction product during the reaction and purification steps. Although no particular limitation is vested on the polymerization inhibitor, use of a phenol-type inhibitor, amine-type inhibitor, mercaptan-type inhibitor or copper powder may generally be mentioned.

As a reaction method, an ordinary reaction vessel may be employed. When using a strong basic substance having a low solubility, the reaction may be effected in accordance with the flow method, namely, by packing the strong basic substance in a column and then causing a mixed solution of the halogen-substituted ether compound and unsaturated amide compound to recirculate through the column. Use of a reaction vessel is however more convenient for the maintenance and control of the reaction apparatus.

The reaction temperature is dependent on the reactivity between each halogen-substituted ether compound and amide compound to be employed. A lower reaction temperature will result in a reduction to the progress of the reaction. On the other hand, a higher reaction temperature will induce side reactions

such as hydrolysis of the amide compound and will hence result in a reduction to the yield of the intended product. Therefore, the reaction is generally carried out within a temperature range of −30—+100°C or preferably −20—+70°C. So long as the reaction is carried out within this temperature range, it may not be absolutely necessaary to maintain the temperature at a constant level during the reaction. It may be possible to change the reaction temperature suitably to allow the reaction to proceed efficiently while precisely observing the progress of the reaction.

Similar to the reaction temperature, the reaction time varies depending on each halogen-substituted ether compound and amide compound to be used. The reaction time is however within 30 hours at the longest, and normally within 20 hours. The progress of the reaction can be observed by depending on changes in nature or state of the reaction system or by determining the concentrations of the raw materials and intended product in the liquid reaction mixture by using gas chromatography or high-speed liquid chromatography.

After the reaction, the intended product of high purity can be obtained by filtering off the by-produced metal halide and distilling the resulting filtrate under reduced pressure in a manner known *per se* in the art. Where the metal halide is dissolved in the liquid reaction mixture, the intended product of high purity can be obtained by driving off the solvent, removing the metal halide with a combination of solvents capable of forming two layers such as hexane-water, benzene-water or chloroform-water, and then distilling the residue under reduced pressure. However, the compounds of this invention generally have high boiling points and many of these compounds have non-volatile properties. In addition, some of them contain readily-polymerizable double bonds in their molecules. Therefore, they may not be purified by distillation in many instances. Even in such cases, the intended products may still be isolated with good purity by solvent extraction or recrystallization or by using an adsorbent such as silica gel, alumina, activated carbon, ion-exchange resin, adsorptive resin or the like. Furthermore, two or more of the above-mentioned purification methods may be used in combination.

Where the reaction solvent has great miscibility with water like dimethylsulfoxide or N,N-dimethylformamide and the intended product is highly hydrophobic, it is feasible to isolate the intended product after the reaction, for example, by adding an aliphatic hydrocarbon solvent such as hexane to the liquid reaction mixture to extract the intended product, adding water to the liquid reaction mixture to separate the intended product as an oil layer, or extracting and separating the intended product with a solvent capable of forming two layers when mixed with water, such as benzene, toluene or chloroform.

According to the present invention, a variety of N-(ω-glycidoxyalkyl)-substituted amide compounds can be prepared using the same reaction procedure and the same reaction reactor. Therefore, the present invention is suitable for the preparation of various products in small volumes.

As shown by the above-exemplified compounds, the N-(ω-glycidoxyalkyl)-substituted amide compounds of this invention are novel compounds obtained by introducing, into the amido groups of amide compounds having various skeletal structures, ω-glycidoxyalkyl groups having reactivity with carboxylic acids or their anhydrides, amines, phenols, thiols and the like. They can be used for various applications depending on the skeletal structures. As specific use, may be mentioned principal raw materials, auxiliary raw materials, crosslinking agents and the like for adhesives, paints, paper-converting or paper-conditioning agents, textile-progessing agents, emulsions, urethane-hardening agents, pigment dispersants, additives to plastics, high-molecular coagulants, ion-exchange resins, adsorption and separation resins, chelate resins, optical lenses, photosensitive resins, soft contact lenses, hygroscopic resins, binders for magnetic materials, binders for composite materials, high-molecular catalysts, clinical reagents, biocompatible materials, enzyme-immobilizing base materials, etc. Namely, the compounds of this invention can be used in a variety of fields.

Since the compound of this invention have such structures in each of which an amido group is contained in a skeletal structure and a glycidyl group is bonded to the amido group by way of a polymethylenoxy group, they have the following advantages.

(i) Since an amido group and a glycidyl group are bonded together via a polymethylenoxy group and the chain length of the polymethylenoxy group can be controlled, flexibility can be imparted to resulting hardened products and the degree of the thus-imparted flexibility can be controlled when the compounds of this invention are used as raw material for hardenable compositions.

Further, when the compounds of this invention are used as raw material for a material for fixing various functional substances, the distance between an amido group in a skeleton and a fixing site of the fixing material can be controlled depending on the molecular size of the substances to be fixed.

(ii) Since an amido group is contained in a skeleton, each of the compounds of this invention has intermolecular secondary bond owing to the hydrogen bond between amido groups. Various properties have thus been improved including physical properties as raw materials.

This invention will next be described further by the following Examples.

## Example 1

Added to 20 ml of N,N-dimethylformamide were 0.72 g of acrylic amide and 1.8 g of 1-bromo-6-glycidoxyhexane. With stirring, 0.46 g of flaky potassium hydroxide which had been ground in a mortar was added. They were reacted at 0—5°C for 6 hours. After that, phenothiazine was added in an amount of 0.5% based on the acrylic amide. After allowing them to react for a predetermined period of time, insoluble

matter was filtered off from the liquid reaction mixture. The solvent and unreacted raw materials were distilled off from the filtrate. The residue was extracted with benzene-water, thereby obtaining the intended product in the benzene layer. Benzene was then driven off from the benzene layer to obtain 1.38 g of N-(6-glycidoxyhexyl)acrylamide (yield: 71%). It was purified further by using column chromatography in which silica gel and benzene were used as an adsorbent and developer respectively. Its melting point was measured and its elementary analysis was also conducted. The following results were obtained. Melting point: 31—32°C. Elementary analysis data: C, 63.82% (Calculated: 63.39%); H, 9.43% (9.32%); N, 6.58% (6.16%).

Examples 2—5

Reactions were carried out under the conditions shown in Table 1, using the combinations of raw materials, strong basic substances and solvents given in Table 1. After the reactions, the respective reaction mixtures were treated in exactly the same manner as in Example 1. Results are shown in Table 2.

**Table 1**

| Ex. | Amide Compound (g) | Halogen-Substituted Ether Compound (g) | Strong Basic Substance (g) | Solvent (ml) | Reaction Temp. (°C) / Reaction Time (hr.) |
|---|---|---|---|---|---|
| 2 | Acrylic amide 0.56 | 1-Bromo-8-glycidoxy-octane 1.6 | KOH 0.32 | DMF 20 | 0–5 / 6 |
| 3 | Acrylic amide 0.52 | 1-Bromo-10-glycidoxy-decane 1.6 | KOH 0.34 | DMF 20 | 0–5 / 6 |
| 4 | Acrylic amide 0.46 | 1-Bromo-12-glycidoxy-dodecane 1.6 | KOH 0.30 | DMF 20 | 0–5 / 6 |
| 5 | p-Hydroxybenz-amide 26.4 | 1-Bromo-4-glycidoxy-butane 80.0 | KOH 46.4 | DMF 800 | 0–5 / 6 |

Note: DMF: N,N-dimethylformamide.

Table 2

| Ex. | Reaction Product | m.p. (°C) | Elementary Analysis Data (%) (Calculated) | | | Yield g, (%) |
|---|---|---|---|---|---|---|
| | | | C | H | N | |
| 2 | N-(8-Glycidoxyoctyl)-acrylamide | 40-41 | 65.60 (65.83) | 9.98 (9.88) | 5.39 (5.48) | 1.11 (72) |
| 3 | N-(10-Glycidoxydecyl)-acrylamide | 38-39 | 67.40 (67.79) | 10.62 (10.33) | 4.79 (4.94) | 1.17 (73) |
| 4 | N-(12-Glycidoxydodecyl)-acrylamide | 60-61 | 69.80 (69.39) | 10.89 (10.69) | 4.94 (4.49) | 1.12 (72) |
| 5 | N-(4-Glycidoxybutyl)-p-(4-glycidoxybutoxy)-benzamide | 27-27 | 63.72 (64.08) | 7.51 (7.95) | 3.48 (3.56) | 56.4 (75) |

Example 6

Added to 20 ml of N,N-dimethylformamide were 1.80 g of acrylic amide and 4.0 g of 1-bromo-4-glycidoxybutane. With stirring, 1.14 g of flaky potassium hydroxide which had been ground in a mortar was added. They were reacted at 0—5°C for 6 hours. After that, phenothiazine was added in an amount of 0.5% based on the acrylic amide. After allowing them to react for a predetermined period of time, insoluble matter was filtered off from the liquid reaction mixture. The solvent and unreacted raw materials were distilled off from the filtrate. The residue was extracted with benzene-water, thereby obtaining the intended product in the benzene layer. Benzene was then driven off from the benzene layer to obtain 3.17 g of N-(4-glycidoxybutyl)acrylamide (yield: 83%). It was purified further by using column chromatography in which silica gel and a mixed benzenemethanol solvent were used as an adsorbent and developer respectively. Its refractive index at 25°C was measured and its elementary analysis was also conducted. The following results were obtained. Refractive index (at 25°C): 1.4885. Elementary analysis data: C, 60.68% (Calculated: 60.26%); H, 8.99% (8.61%); N, 7.18% (7.03%).

Examples 7—13

Reactions were carried out under the conditions shown in Table 3, using the combinations of raw materials, strong basic substances and solvents given in Table 3. After the reactions, the respective reaction mixtures were treated in exactly the same manner as in Example 6. Results are shown in Table 4.

Table 3

| Ex. | Amide Compound (g) | Halogen-Substituted Ether Compound (g) | Strong Basic Substance (g) | Solvent (ml) | Reaction Temp. (°C) / Reaction Time (hr.) |
|---|---|---|---|---|---|
| 7 | Methacrylic amide 1.08 | 1-Bromo-4-glycidoxy-butane 2.0 | KOH 0.56 | DMF 20 | 0-5 / 6 |
| 8 | Salicylic amide 0.66 | 1-Bromo-4-glycidoxy-butane 2.0 | KOH 1.12 | DMF 20 | 0-5 / 6 |
| 9 | Benzamide 1.16 | 1-Bromo-4-glycidoxy-butane 2.0 | KOH 0.56 | DMF 20 | 0-5 / 6 |
| 10 | Lactic amide 0.42 | 1-Bromo-4-glycidoxy-butane 2.0 | KOH 1.08 | DMF 20 | 0-5 / 6 |
| 11 | β-Hydroxy-propionamide 0.42 | 1-Bromo-4-glycidoxy-butane 2.0 | KOH 1.08 | DMF 20 | 0-5 / 6 |
| 12 | Isophthalic amide 0.78 | 1-Bromo-4-glycidoxy-butane 2.0 | NaOH 0.80 | DMSO 20 | 20-30 / 6 |
| 13 | Terephthalic amide 0.78 | 1-Bromo-4-glycidoxy-butane 2.0 | NaOH 0.80 | DMSO 20 | 20-30 / 6 |

Note: DMSO: dimethylsulfoxide.

Table 4

| Ex. | Reaction Product | Refractive index (25°C) | Elementary Analysis Data (%) (Calculated) | | | Yield g, (%) |
|---|---|---|---|---|---|---|
| | | | C | H | N | |
| 7 | N-(4-Glycidoxybutyl)-methacrylamide | 1.4828 | 62.38 (61.93) | 8.88 (8.99) | 6.37 (6.56) | 1.59 (78) |
| 8 | N-(4-Glycidoxybutyl)-o-(4-glycidoxybutoxy)-benzamide | 1.5265 | 63.39 (64.08) | 8.37 (7.95) | 3.36 (3.56) | 1.43 (71) |
| 9 | N-(4-Glycidoxybutyl)-benzamide | 1.5305 | 66.45 (67.43) | 7.67 (7.69) | 5.04 (5.61) | 2.05 (86) |
| 10 | N-(4-Glycidoxybutyl)-α-(4-glycidoxybutoxy)-propionamide | 1.4721 | 60.35 (59.09) | 9.88 (9.06) | 3.44 (4.05) | 1.16 (70) |
| 11 | N-(4-Glycidoxybutyl)-β-(4-glycidoxybutoxy)-propionamide | 1.4638 | 59.76 (59.09) | 9.72 (9.06) | 3.85 (4.05) | 1.19 (72) |
| 12 | N,N'-Di(4-glycidoxy-butyl)isophthalamide | 1.5181 | 63.43 (62.82) | 7.78 (7.68) | 6.27 (6.66) | 1.53 (76) |
| 13 | N,N'-Di(4-glycidoxy-butyl)terephthalamide | 1.4945 | 63.24 (62.82) | 8.29 (7.68) | 5.82 (6.66) | 1.39 (69) |

Example 14

Using N-(4-glycidoxybutyl)-p-(4-glycidoxybutoxy)benzamide prepared in Example 5, an adhesion test of steel plates was conducted with the following formulation.

Formulation:

Thoroughly kneaded in a three-roll mill were 100 parts of N-(4-glycidoxybutyl)-p-(4-glycidoxybutoxy)-benzamide, 4 parts of dicyandiamide and 2 parts of aerosil.

In addition, 30 parts of alumina were also uniformly dispersed in the resultant mixture. The thus-obtained mixture was then deaerated under reduced pressure to obtain a formulation.

Preparation of specimen:

The above formulation was coated into a steel plate (JIS G3141), which had been subjected to a degreasing treatment with acetone and has dimensions of 25 mm wide, 100 mm long and 1.6 mm thick, to an area 12.5 mm apart from one end thereof. Then, another steel plate was superposed on the thus-coated steel plate. While pressing the plates with clips, it was hardened at 180°C for 60 minutes to provide a specimen.

Test:

The tensile shear strength of the specimen was measured in accordance with JIS K6850. It was found to be 160 kg/cm$^2$.

Another specimen was also prepared and tested in the same manner as described above except for the use of Epicoat 828 of the bisphenol A type (product of Yuka-Shell Epoxy K.K.) instead of N-(4-glycidoxybutyl)-p-(4-glycidoxybutoxy)benzamide. This test gave a value of 120 kg/cm$^2$.

Example 15

A formulation was obtained in exactly the same manner as in Example 14 except that N-(4-glycidoxybutyl)-p-(4-glycidoxybutoxy)benzamide was used. A specimen was prepared using the thus-obtained formulation. Its peel strength test was measured.

Preparation of specimen:

The above formulation was coated onto a steel plate (JIS G3141), which had been subjected to a degreasing treatment with acetone and had dimensions of 25 mm wide, 100 mm long and 0.5 mm thick, to an area 70 mm apart from one end thereof. Then, another steel plate was superposed on the thus-coated steel plate. While pressing the plates with clips, it was hardened at 180°C for 60 minutes to provide a specimen.

Test:

Following JIS K6854, the peel strength of the specimen was measured. It was found to be 35 kg/25 mm. Furthermore, another specimen was prepared and tested in the same manner as described above except for the employment of Epicoat 828 of the bisphenol A type (product of Yuka-Shell Epoxy K.K.) instead of N-(4-glycidoxybutyl)-p-(4-glycidoxybutoxy)benzamide. As a result, a value of 3 kg/25 mm was obtained.

**Claims**

1. An N-(ω-glycidoxyalkyl)-substituted amide compound represented by the following general formula:

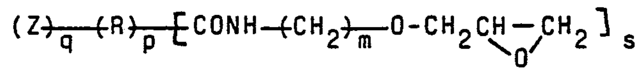

(1a)

or

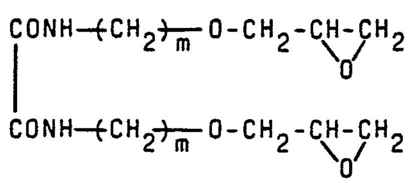

(1b)

wherein R means an aliphatic, aromatic or alicyclic hydrocarbon group, Z denotes a hydrogen atom or an ω-glycidoxyalkoxy or N,N-di(ω-glycidoxyalkyl)amino group, p stands for an integer of 0 or 1, q is an integer of 1—4, s is an integer of 1—4 and m is an integer of 4—20, with the provisos that: (i) q is 1, Z is H and s is 1 when p = 0, and (ii) p is 1 when Z denotes an ω-glycidoxyalkoxy or N,N-di(ω-glycidoxyalkyl)amino group.

2. The compound as claimed in Claim 1, wherein the N-(ω-glycidoxyalkyl)-substituted amide compound is:

an N-(ω-glycidoxyalkyl)-substituted amide compound of a mono-amide compound;

an N-(ω-glycidoxyalkyl)-substituted amide compound of a polyamide compound including diamide compound;

an N-(ω-glycidoxyalkyl)-substituted ω-glycidoxyalkyl ether compound of a hydroxyl-substituted monoamide compound;

an N-(ω-glycidoxyalkyl)-substituted ω-glycidoxyalkyl ether compound of a hydroxyl-substituted diamide compound;

an N-(ω-glycidoxyalkyl)-substituted amide compound of an amino-substituted monoamide compound; or

an N-(ω-glycidoxyalkyl)-substituted amide compound of an amino-substituted diamide compound.

3. The compound as claimed in Claim 2, wherein the N-(ω-glycidoxyalkyl)-substituted amide compound of the monoamide compound is selected from:

N-(4-glycidoxydibutyl)acrylamide;
N-(6-glycidoxyhexyl)acrylamide;
N-(8-glycidoxyoctyl)acrylamide;
N-(10-glycidoxydecyl)acrylamide;
N-(12-glycidoxydodecyl)acrylamide;
N-(4-glycidoxybutyl)methacrylamide; or
N-(4-glycidoxybutyl)benzamide.

4. The compound as claimed in Claim 2, wherein the N-(ω-glycidoxyalkyl)-substituted ω-glycidoxyalkylether compound of the hydroxyl-substituted monoamide compound is selected from:

N-(4-glycidoxybutyl)-p-(4-glycidoxybutoxy)benzamide;
N-(4-glycidoxybutyl)-o-(4-glycidoxybutoxy)benzamide;
N-(4-glycidoxybutyl)-α-(4-glycidoxybutoxy)propionamide; or
N-(4-glycidoxybutyl)-β-(4-glycidoxybutoxy)propionamide.

5. The compound as claimed in Claim 2, wherein the N-(ω-glycidoxyalkyl)-substituted amide compound of the diamide compound is:

N,N′-di(4-glycidoxybutyl)isophthalamide; or
N,N′-di(4-glycidoxybutyl)terephthalamide.

6. A process for preparing an N-(ω-glycidoxyalkyl)-substituted amide compound of claim 1, which comprises reacting an amide compound represented by the following general formula:

$$(Y)_q-(R)_p-(CONH_2)_s \qquad (2)$$

wherein Y denotes a hydrogen atom or a hydroxyl or amino group and R is as defined in claim 1, p = q = 0, and s = 2; or R, q, p, and s are as defined in claim 1 with the proviso that q is 1, Y is H and s is 1 when p is 0, with a halogen-substituted glycidyl ether compound represented by the following general formula:

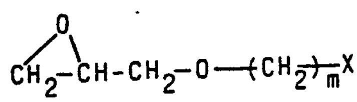
(3)

wherein X means a halogen atom and m has the same meaning as in claim 1, in the presence of a strong basic substance in an aprotic polar solvent.

7. The process as claimed in Claim 6, wherein the halogen-substituted glycidyl ether compound is used in a molar amount 0.2—20 times the amide compound.

8. The process as claimed in Claim 6, wherein the strong basic substance is used in a molar amount 0.2—15 times the amide compound.

9. The process as claimed in any one of claims 6, 7 and 8, wherein the halogen-substituted glycidyl ether compound and amide compound are reacted at −30°C to +100°C.

**Patentansprüche**

1. N-(ω-Glycidoxyalkyl)-substituierte Amid-Verbindung, dargestellt durch die folgende allgemeine Formel:

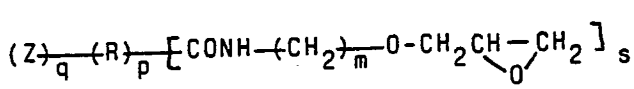
(1a)

oder

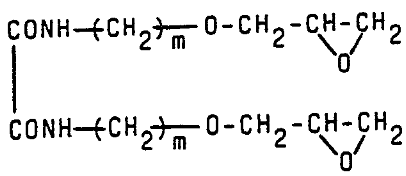
(1b)

worin bedeuten:

R eine aliphatische, aromatische oder alicyclische Kohlenwasserstoffgruppe, Z ein Wasserstoffatom oder eine ω-Glycidoxyalkoxy- oder N, N-Di-(ω-glycidoxyalkyl)aminogruppe, p die ganze Zahl 0 oder 1, q eine ganze Zahl von 1 bis 4, s eine ganze Zahl von 1 bis 4 und m eine ganze Zahl von 4 bis 20, mit der Maßgabe, daß (i) q = 1, Z = H und s = 1, wenn p = 0, und (ii) p = 1, wenn Z eine ω-Glycidoxyalkoxy- oder N,N-Di-(ω-glycidoxyalkyl)aminogruppe bedeutet.

2. Verbindung nach Anspruch 1, worin die N-(ω-Glycidoxyalkyl)-substituierte Amidverbindung ist:

eine N-(ω-Glycidoxyalkyl)-substituierte Amidverbindung einer Monoamidverbindung;

eine N-(ω-Glycidoxyalkyl)-substituierte Amidverbindung einer Polyamidverbindung einschließlich einer Diamidverbindung;

eine N-(ω-Glycidoxyalkyl)-substituierte ω-Glycidoxyalkylätherverbindung einer Hydroxyl-substituierten Monoamidverbindung;

eine N-(ω-Glycidoxyalkyl)-substituierte ω-Glycidoxyalkylätherverbindung einer Hydroxyl-substituierten Diamidverbindung;

eine N-(ω-Glycidoxyalkyl)-substituierte Amidverbindung einer Amino-substituierten Monoamidverbindung; oder

eine N-(ω-Glycidoxyalkyl)-substituierte Amidverbindung einer Amino-substituierten Diamidverbindung.

3. Verbindung nach Anspruch 2, worin die N-(ω-Glycidoxyalkyl)-substituierte Amidverbindung der Monoamidverbindung ausgewählt wird aus:

N-(4-Glycidoxydibutyl)acrylamid;
N-(6-Glycidoxyhexyl)acrylamid;
N-(8-Glycidoxyoctyl)acrylamid;
N-(10-Glycidoxydecyl)acrylamid;
N-(12-Glycidoxydodecyl)acrylamid;
N-(4-Glycidoxybutyl)methacrylamid) oder
N-(4-Glycidoxybutyl)benzamid.

4. Verbindung nach Anspruch 2, worin die N-(ω-Glycidoxyalkyl)-substituiert ω-Glycidoxyalkylätherverbindung der Hydroxyl-substituierten Monoamidverbindung ausgewählt wird aus:

N-(4-Glycidoxybutyl)-p-(4-glycidoxybutoxy)benzamid;
N-(4-Glycidoxybutyl-o-(4-glycidoxybutoxy)benzamid;
N-(4-Glycidoxybutyl)-α-(4-glycidoxybutoxy)propionamid; oder
N-(4-Glycidoxybutyl)-β-(4-glycidoxybutoxy)propionamid.

5. Verbindung nach Anspruch 2, worin die N-(ω-Glycidoxyalkyl)-substituiert Amidverbindung der Diamidverbindung ist:

N,N-Di-(4-glycidoxybutyl)isophthalamid; oder
N,N'-Di-(4-glycidoxybutyl)terephthalamid.

6. Verfahren zur Herstellung einer N-(ω-Glycidoxyalkyl)substituierten Amidverbindung nach Anspruch 1, das umfaßt die Umsetzung einer Amidverbindung der nachstehend angegebenen allgemeinen Formel:

$$(Y)_q-(R)_p-(CONH_2)_s \qquad (2)$$

worin Y ein Wasserstoffatom oder eine Hydroxyl- oder Aminogruppe darstellt und R wei in Anspruch 1 definiert ist, p = q = 0 und s = 2; oder worin R, q, p und s wie in Anspruch 1 definiert sind, mit der Maßgabe, daß p = 1, Y = H und s = 1, wenn p = 0; mit einer Halogen-substituierten Glycidylätherverbindung der nachstehend angegebenen allgemeinen Formel:

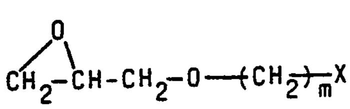

(3)

worin X ein Halogenatom bedeutet und m die gleiche Bedeutung wie in Anspruch 1 hat, in Gegenwart einer stark basischen Substanz in einem aprotischen polaren Lösungsmittel.

7. Verfahren nach Anspruch 6, worin die Halogen-substituierte Glycidylätherverbindung in einer Molmenge verwendet wird, die dem 0,2- bis 20-fachen der Amidverbindung entspricht.

8. Verfahren nach Anspruch 6, worn die stark basische Substanz in einer Molmenge verwendet wird, die dem 0,2-bis 15-fachen der Amidverbindung entspricht.

9. Verfahren nach einem der Ansprüche 6, 7 und 8, worin die Halogen-substituierte Glycidylätherverbindung und die Amidverbindung bei −30°C bis +100°C miteinander umgesetzt werden.

**Revendications**

1. Composé amide N-(ω-glycidoxyalkyl)-suibstitué représenté par la formule générale suivante:

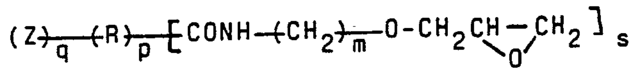

(1a)

ou

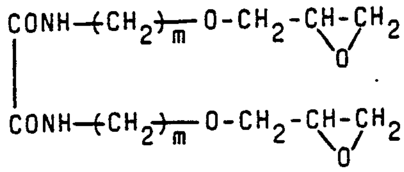

(1b)

où R indique un groupe hydrocarbure aliphatique, aromatique ou alicyclique, Z désigne un atome d'hydrogène ou un groupe ω-glycidoxyalcoxy ou N,N-di(ω-glycidoxyalkyl)amino, p indique un nombre entier 0 ou 1, q est un nombre entier de 1—4, s est un nombre entier de 1—4 et m est un nombre entier de 4—20, à conditions que: (i) q soit 1, Z soit H et s soit 1 quand p=0 et (ii) p soit 1 quand Z désigne un groupe ω-glycidoxyalcoxy ou N,N-di(ω-glycidoxyalkyl)amino.

2. Composé selon la revendication 1, où le composé amide N-(ω-glycidoxyalkyl)-substitué est
un composé amide N-(ω-glycidoxyalkyl)-substitué d'un composé monoamide;
un composé amide N-(ω-glycidoxyalkyl)-substitué d'un composé polyamide comprenant un composé diamide;
un composé ω-glycidoxyalkyl éther N-(ω-glycidoxyalkyl)-substitué d'un composé monoamide hydroxylesubstitué;
un composé ω-glycidoxyalkyl éther N-(ω-glycidoxyalkyl)-substitué d'un composé diamide hydroxylesubstitué;
un composé amide N-(ω-glycidoxyalkyl)-substitué d'un composé monoamide amino-substitué; ou
un composé amide N-(ω-glycidoxyalkyl)-substitué d'un composé diamide amino-substitué.

3. Composé de la revendication 2, où le composé amide N-(ω-glycidoxyalkyl)-substitué du composé monoamide est choisi parmi:
N-(4-glycidoxybutyl)acrylamide;
N-(6-glycidoxyhexyl)acrylamide;
N-(8-glycidoxyoctyl)acrylamide;
N-(10-glycidoxydécyl)acrylamide;
N-(12-glycidoxydodécyl)acrylamide;
N-(4-glycidoxybutyl)méthacrylamide; ou
N-(4-glycidoxybutyl)benzamide.

4. Composé de la revendication 2, où le composé ω-glycidoxyalkyl éther N-(ω-glycidoxyalkyl)-substitué du composé monoamide hydroxyle-substitué est choisi parmi:
N-(4-glycidoxybutyl)-p-(4-glycidoxybutoxy)benzamide;
N-(4-glycidoxybutyl)-o-(4-glycidoxybutoxy)benzamide;
N-(4-glycidoxybutyl)-α-(4-glycidoxybutoxy)propionamide; ou
N-(4-glycidoxybutyl)-β-(4-glycidoxybutoxy)propionamide.

5. Composé de la revendication 2, où le composé amide N-(ω-glycidoxyalkyl)-substitué de composé diamide est:
N,N'-di(4-glycidoxybutyl)isophtalamide; ou
N,N'-di(4-glycidoxybutyl)téréphtalamide.

6. Procédé de préparation d'un composé amide N-(ω-glycidoxyalkyl)-substitué de la revendication 1, qui comprend la réaction d'un composé amide représenté par la formule générale:

$$(Y)_q\text{—}(R)_p\text{—}(CONH_2)_s \qquad (2)$$

où Y désigne un atome d'hydrogène ou un groupe hydoxyle ou amino et R est tel que défini à la revendication 1, p = q = 0 et s = 2; ou bien R, q, p et s sont tels que définis à la revendication 1 à condition que q soit 1, Y soit H et s soit 1 quand p = 0; avec un composé glycidyl éther halogéno-substitué représenté par la formule générale suivante:

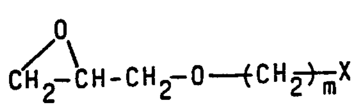

(3)

où X indique un atome d'halogène et m a la même signification qu'à la revendication 1, en présence d'une substance basique forte dans un solvant aprotique polaire.

7. Procédé selon la revendication 6, où le composé glycidyl éther halogéno-substitué est utilisé en une quantité molaire de 0,2—20 fois le composé amide.

8. Procédé selon la revendication 6, où la substance basique forte est utilisée en une quantité molaire de 0,2 à 15 fois le composé amide.

9. Procédé selon l'une quelconque des revendications 6, 7 et 8, où on fait réagir le composé glycidyl éther halogéno-substitué et le composé amide entre −30°C et +100°C.